# Europäisches Patentamt

(19) **European Patent Office**

**Office européen des brevets**

(11) Publication number: **0 230 153**
**B1**

(12) **EUROPEAN PATENT SPECIFICATION**

(45) Date of publication of the patent specification:
**27.12.89**

(51) Int. Cl.⁴: **A 61 N 1/30**

(21) Application number: **86310230.7**

(22) Date of filling: **31.12.86**

(54) **A low-frequency therapeutic device for iontophoresing cation and anion.**

(30) Priority: **31.12.85 JP 298535/85**

(43) Date of publication of application:
**29.07.87 Bulletin 87/31**

(45) Publication of the grant of the patent:
**27.12.89 Bulletin 89/52**

(84) Designated Contracting States:
**DE FR GB**

(56) References cited:
**WO-A-85/03449
AT-B-364 722
DE-A-2 222 844
FR-A-2 563 437
US-A-4 406 658
US-A-4 457 748**

(73) Proprietor: **Hayashibara, Ken, 9-8, 4-chome,
Higashi- Furumatsu, Okayama- shi Okayama (JP)**

(72) Inventor: **Masaki, Kazumi, 7-3, 4-chome,
Fujishirodai, Suita- shi Osaka (JP)**

(74) Representative: **Palmer, Roger, PAGE, WHITE &
FARRER 54 Doughty Street, London WC1N 2LS
(GB)**

EP 0 230 153 B1

LIBER, STOCKHOLM 1989

## Description

The present invention relates to a low-frequency therapeutic device for iontophoresing the cationic and anionic elements of an ionic medicament.

Iontophoresis of a liquid medicine may dramatically increase its efficacy. When the medicine contains cationic and anionic elements, penetration of both elements may further augment the efficacy.

In the case of certain medicaments, for example, "NK 343 (6-[2-[5-bromo-2-pyridyl) amino]vinyl]-1-ethyl-2-picolinium iodide)", an aminovinyl photosensitizing dye commercialized by Nippon Kankoh-Shikiso Kenkyusho Co., Ltd., Okayama, Japan, the therapeutic efficacy on growth and regeneration of hair is augmented to some extent by iontophoresis of the cationic element, but extremely by iontophoresis of both cationic and anionic elements.

However, conventional therapeutic devices cannot be used for this purpose because the circuit must be preselected in accordance with the polarity of the ionic element to be penetrated. US-A-4 406 658 discloses an iontophoretic device in which the polarity of the electrodes is reversible.

It is an object of the present invention to provide a low-frequency therapeutic device having a polarity selection function such that the whole of an ionized medicament can be adequately penetrated into the deeper part of the skin, regardless of the polarity of the elements of the medicament.

According to the present invention there is provided a low-frequency therapeutic device for iontophoresing the cationic and anionic elements of an inoic medicament, comprising:

means for generating a low-frequency voltage;
means for selecting the polarity of said low-frequency voltage, said selecting means being operable in association with the on/off operation of a switch in the power supply circuit including a power source; and
active electrode means connected by way of said selecting means to the output of said generating means.

Embodiments of the present invention will hereinafter be described, by way of example, with reference to the accompanying drawings, in which:

FIG. 1  shows the circuit of an embodiment of a therapeutic device of the invention and FIG. 2 illustrates its operation;
FIG. 3  shows the circuit of another embodiment of the device of the invention and FIG. 4 illustrates its operation; and
FIG. 5  shows the circuit of a still further embodiment of a therapeutic device of the invention and FIG. 6 illustrates its operation.

Throughout the accompanying drawings, reference B is used to denote a battery; A is a low-frequency generator; E and F are active electrodes; $S_1$ to $S_3$ are rotary switches; and reference numerals 1 to 8 denote contacts or selection positions of a rotary switch.

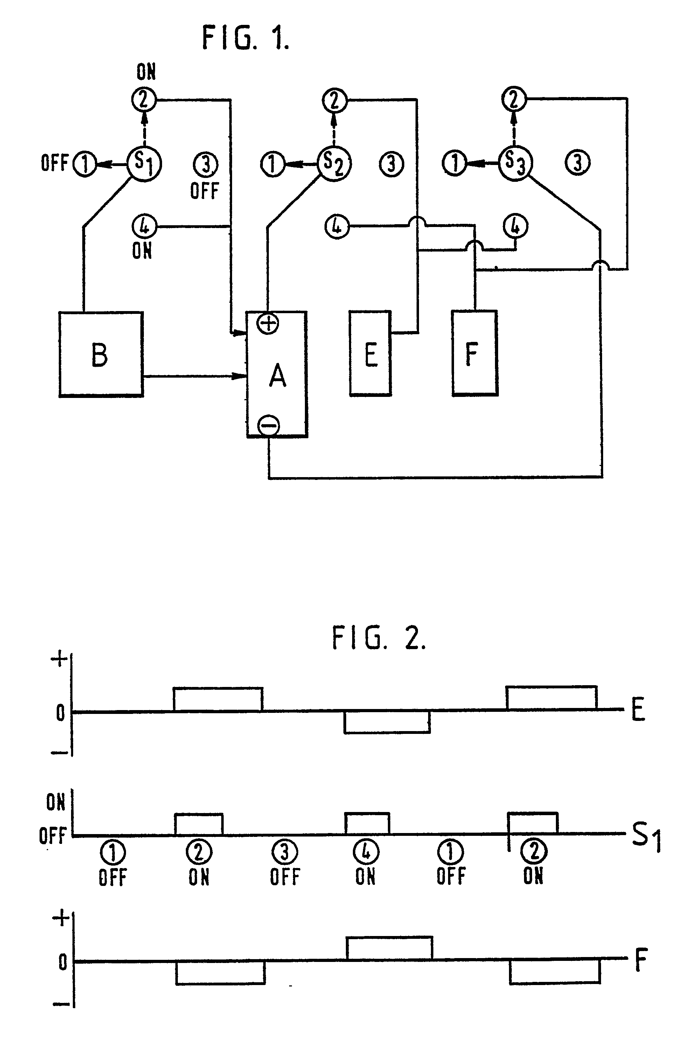

In the circuit of the therapeutic device shown in FIG. 1, when a switch $S_1$ is in its position 1, the circuit is off and no current is supplied to a low-frequency generator A. When the switch $S_1$ is rotated to its position 2, as indicated by a broken line, the circuit is closed and the low-frequency generator A is connected to the supply.

In this case, switches $S_2$ and $S_3$ are also in their positions 2 because they are associated with the switch $S_1$. The low-frequency voltage from the plus terminal of the low-frequency generator A is applied to an active electrode E by way of contact 2 of the switch $S_2$, whilst the low-frequency voltage from the minus terminal of the low-frequency generator A is applied to an active electrode F by way of contact 2 of the switch $S_3$.

When the switch $S_1$ is rotated to its position 3, the circuit is off. When the switch $S_1$ is rotated further to its position 4, the circuit is closed and the battery is once again connected to the generator A. However, in this position, the current across active electrodes E and F is reversed in direction as compared to that when the rotary switches are in their position 2.

Thus, the positive and negative low-frequency voltages energize by turns active electrodes E and F whenever on/off operation of the power switch $S_1$ is effected.

FIG. 2 illustrates the relationship between the contact positions of the rotary switches and the voltage supplied to the active electrodes E and F.

As is clear from this figure, since the polarity of the voltage at the active electrodes E and F can be switched in conjunction with the on/off operation of the power source, the whole of a medicament can be adequately penetrated, regardless of the polarity of the elements.

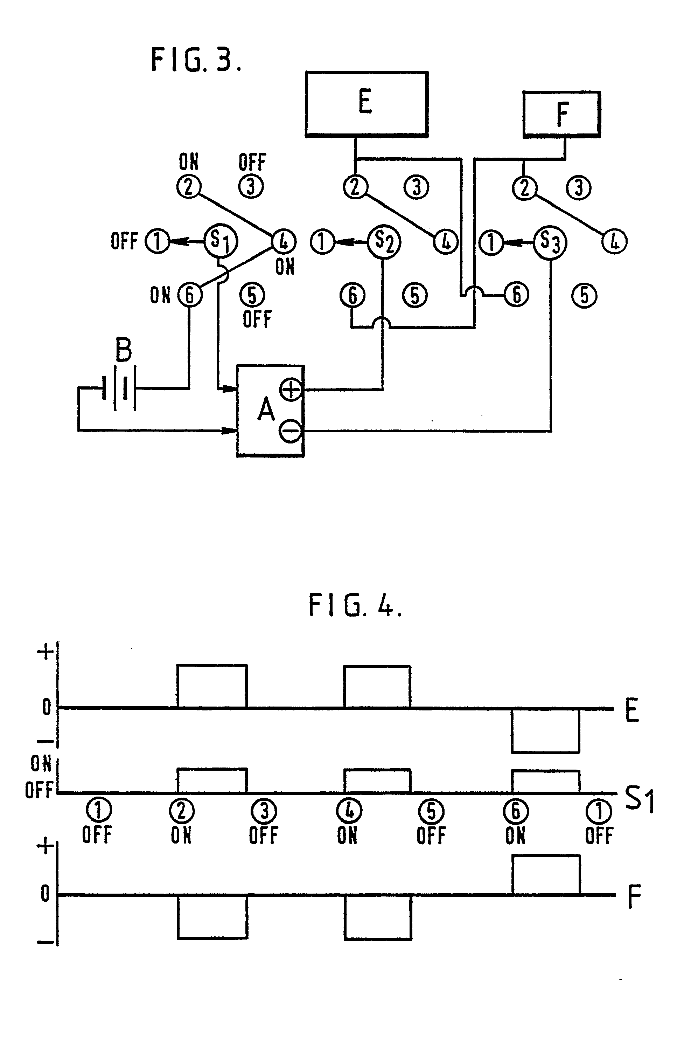

FIG. 3 shows the circuit of another embodiment of a therapeutic device of the invention, in which switches $S_1$, $S_2$ and $S_3$ respectively have six selections and two of the three ON selections are electrically equivalent. This embodiment differs from the preceding embodiment in this respect. With this arrangement, the polarity of the voltage at the active electrodes E and F is not switched at the first or second on/off operation, but is changed at the third on/off operation. More particularly, the active electrodes E and F are energized with the positive and negative voltages at the selection ratio of 2 : 1.

In this way, the cationic and anionic elements in a liquid medicine can be equally penetrated into the deeper part of the skin even when the elements are different, for example, in skin-permeability.

FIG. 4 illustrates the relationship between the contact positions of the rotary switches in the circuit shown in FIG. 3 and the voltage supplied to the active electrodes E and F.

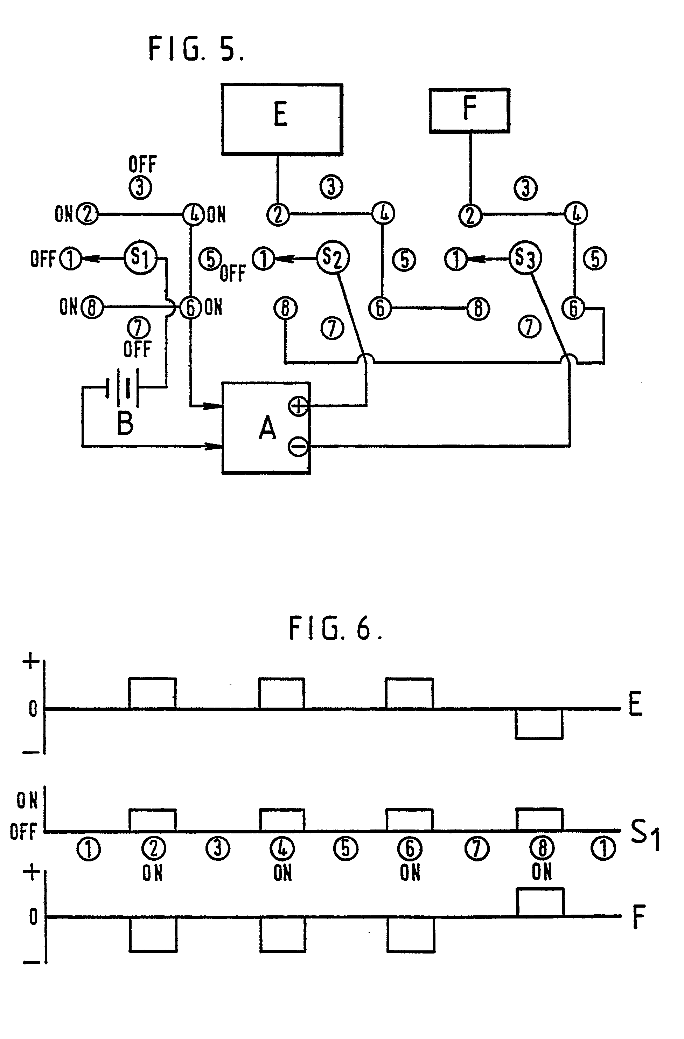

FIG. 5 shows the circuit of a still further embodiment of a device of the invention, in which three out of four ON selections in switches $S_2$ and $S_3$ are electrically equivalent.

With this arrangement, the polarity of the voltage supplied to the active electrodes E and F is switchable at each fourth on operation of the power source. In this case, the selection ratio is 3 : 1, and this ratio can be used with advantage to cause penetration of the cationic and anionic elements in a liquid medicine where these elements differ substantially in skin-permeability.

FIG. 6 illustrates the relationship between the contact positions of the rotary switch in the circuit shown in FIG. 5 and the voltage supplied to the active electrodes.

In the above embodiments the use of the selection ratios 1 : 1, 2 : 1 and 3 : 1 has been disclosed. However, the selection ratio can be chosen according to the type, skin-permeability and/or dosage of ionic elements. It is possible to enable the selection ratio to be freely selected by providing the therapeutic device with a suitable selection switch.

By equipping the rotary switch with a suitable reverse-rotation stopping means, the cationic and anionic elements of a medicament can be much more adequately penetrated.

As described above, the efficacy that is hardly achievable by iontophoresis of either ionic element is possible by causing penetration of the cationic and anionic elements using a device of the invention.

Since the duration of the positive or negative voltage can be automatically selected in accordance with the type and dosage of the medicament to be used, it is not necessary in a device of the invention to preset the duration by a complicated manual switching operation.

**Claims:**

1. A low-frequency therapeutic device for iontophoresing the cationic and anionic elements of an ionic medicament, comprising:

means (A) for generating a low-frequency voltage;
means ($S_2$, $S_3$) for selecting the polarity of said low-frequency voltage, said selecting means being operable in association with the on/off operation of a switch ($S_1$) in the power supply circuit including a power source (B); and active electrode means (E, F) connected by way of said selecting means ($S_2$, $S_3$) to the output of said generating means (A).

2. A therapeutic device as claimed in Claim 1, wherein said selecting means is a rotary switch ($S_2$).

3. A therapeutic device as claimed in Claim 1 or 2, wherein one selection cycle consists of two selections and the output polarity is reversed on each on/off operation of the power source.

4. A therapeutic device as claimed in Claim 1 or 2, wherein one selection cycle consists of three selections and the output polarity is reversed on each third on/off operation of the power source.

5. A therapeutic device as claimed in Claim 1 or 2, wherein one selection cycle consists of four selections and the output polarity is reversed on each fourth on/off operation of the power source.

**Patentansprüche**

1. Niederfrequenz-Therapiegerät zur Iontophorese der kathionischen und anionischen Elemente eines ionischen Medikamentes, das eine Einrichtung (A) zur Erzeugung einer Niederfrequenzspannung aufweist; eine Einrichtung ($S_2$, $S_3$) um die Polarität dieser Niederfrequenzspannung auszuwählen, wobei diese Auswähleinrichtung mit dem Ein-/Aus-Schaltvorgang eines Schalters ($S_1$) in er Stromversorgungsschaltung, die eine Spannungsquelle (B) enthält, zusammenwirkt; und Elektrodenmittel (E, F), die über die Auswähleinrichtung ($S_2$, $S_3$) mit dem Ausgang der Erzeugungseinrichtung (A) verbunden sind.

2. Therapiegerät nach Anspruch 1 bei dem die Auswähleinrichtung als Drehschalter ($S_2$, $S_3$) ausgebildet ist.

3. Therapiegerät nach Anspruch 1 oder 2, bei dem ein Auswählzyklus aus zwei Auswählvorgängen besteht und die Ausgangspolarität bei jedem An-/Aus-Schaltvorgang der Spannungsquelle umgekehrt wird.

4. Therapiegerät nach Anspruch 1 oder 2, bei dem ein Auswählzyklus aus drei Auswählvorgängen besteht und die Ausgangspolarität bei jedem dritten Ein-/Aus-Schalvorgang der Spannungsquelle umgekehrt wird.

5. Therapiegerät nach Anspruch 1 oder 2, bei dem ein Auswählzyklus aus drei Auswählvorgängen besteht und die Ausgangspolarität bei jedem dritten Ein-/Aus-Schaltvorgang der Spannungsquelle umgekehrt wird.

**Revendications**

1. Dispositif thérapeutique à basse fréquence en vue de l'iontophorèse des éléments cationiques et anioniques d'un médicament ionique comportant des moyens (A) pour générer une tension à basse fréquence;

des moyens ($S_2$, $S_3$) en vue de sélectionner la polarité de ladite tension à basse fréquence, lesdits moyens de sélection étant actionnables en association avec le fonctionnement marche/arrêt d'un interrupteur ($S_1$) dans le circuit d'alimentation comprenant une source d'énergie (B); et
des électrodes actives (E, F) reliées à l'aide desdits moyens de sélection (S2, S3) à la sortie desdits moyens générateurs (A).

2. Dispositif thérapeutique selon la revendication 1, dans lequel lesdits moyens de sélection sont un commutateur rotatif (S2, S3).

3. Dispositif thérapeutique selon la revendication 1 ou 2, dans lequel un cycle de sélection consiste en deux sélections et la polarité de sortie est inversée lors de chaque manoeuvre marche/arrêt de la source d'alimentation.

4. Dispositif thérapeutique selon la revendication ou 2, dans lequel un cycle de sélection consiste en trois sélections et la polarité de sortie est inversée lors de chaque troisième manoeuvre marche/arrêt de la source d'alimentation.

5. Dispositif thérapeutique selon la revendication 1 ou 2, dans lequel un cycle de sélection consiste en quatre sélections et la polarité de sortie est inversée lors de chaque quatrième manoeuvre marche/arrêt de la source d'alimentation.

# FIG. 1.

# FIG. 2.

# FIG. 3.

# FIG. 4.

# FIG. 5.

# FIG. 6.